(19) Europäisches Patentamt
European Patent Office
Office européen des brevets

(11) Publication number: 0 231 420 B1

(12) # EUROPEAN PATENT SPECIFICATION

(45) Date of publication of patent specification: **04.09.91** (51) Int. Cl.$^5$: **A61K 6/10**

(21) Application number: **86101537.8**

(22) Date of filing: **06.02.86**

(54) **Dental impression material.**

(43) Date of publication of application:
**12.08.87 Bulletin 87/33**

(45) Publication of the grant of the patent:
**04.09.91 Bulletin 91/36**

(84) Designated Contracting States:
**AT BE CH DE FR GB IT LI LU NL SE**

(56) References cited:
**DE-B- 2 630 393**
**FR-A- 2 185 006**
**FR-A- 2 429 810**
**US-A- 3 950 300**
**US-A- 4 477 626**
**US-A- 4 537 944**

(73) Proprietor: **DENTSPLY GMBH**
**Reichenaustrasse 150**
**W-7750 Konstanz 12(DE)**

(72) Inventor: **Gribi, Hanspeter K., Dr.**
**Bifrangstrasse 7**
**CH-8730 Uznach(CH)**

(74) Representative: **Wächtershäuser, Günter, Dr.**
**Tal 29**
**W-8000 München 2(DE)**

EP 0 231 420 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The invention refers to silicone rubber dental impression materials. These dental impression materials are used for reproduction of hard tissue, i.e. dental enamel, and for crown and bridge purposes, as well as for registering the soft tissue of the mouth. They are either of the condensation type which are cured by a condensation reaction of an organic silicate with a hydroxy terminated silicone, or of the addition type which are cured by the reaction of a vinyl terminated silicone with a hydride-containing silicone. Addition type silicones are the more dimensionally stable and accurate of the two classes of silicones.

A major disadvantage of the silicone type materials resides in their hydrophobic nature by which they are not readily wet upon coming into contact with hydrophilic surfaces, for example the soft tissues of the mouth or by wet plaque upon hard tissues, e.g. enamel surfaces. Likewise the hydrophobic silicones are not easily wet by mixes of calcium sulfate hemihydrate and water, and yet it is almost universal that impressions of the crown and bridge type are poured and reproduced using calcium sulfate hemihydrate and water, whereafter the mixture is allowed to harden to form plaster of paris within the impression mold. The consequence of this is that voids are sometimes found on the surfaces adjacent to the impression. Therefore it is very desirable to improve both the wetting properties of the set silicone into which is poured the plaster of paris, and the wetting properties of the pre-set silicone when it is in contact with hydrophilic surfaces such as plaque overlaid hard tissue, gingiva or other soft tissue.

One procedure for measuring the ability to wet a substrate or to be wet by it is known as the contact angle method. In such a procedure a single drop of water is placed on the surface of the material to be tested and, depending on the angle the water forms with the surface upon which it is placed in contact, greater or lesser tendency to wet that surface is noted. Thus, very hydrophilic surfaces would have low contact angle, with the drop spreading over the surface. On the other hand, the completely hydrophobic silicone materials have a contact angle of 90° or more.

It has been attempted to improve the wettability of silicone impression materials by the incorporation of nonylphenoxy-poly(ethyleneoxy)ethanols (K.B. Norling, M.H. Reisbick, The Journal of Prosthetic Dentistry 42, 342-347 (1979)). With some of these additives, the wetting angle is indeed reduced, but only to values of about 40°. Thus, the disadvantageous effects of a low wettability are not sufficiently avoided.

Further, the increased wettability is associated with a concomitant increased degree of water sorption. Excessive water sorption leads to dimensional instability. This is a highly objectionable side effect of the previous attempt to improve the wettability of silicone impression materials.

US-A-4 477 626 describes a curable liquid composition comprising a vinyl-containing polyorganosiloxane, a polyorganosiloxane containing at least two silicon-bonded hydrogen atoms per molecule, a platinum-containing catalyst and a finely divided silica filler by including in said composition from 0.05 to 10 %, based on the weight of said composition, of a polyorganosiloxane wherein each molecule contains at least 0.5 % by weight of hydroxyl groups.

FR-A-2 429 810 discloses a curable composition comprising (a) an organopolysiloxane having at least two silicon-bound vinyl groups per molecule, (b) an organohydrogenpolysiloxane containing at least two silicon-bound hydrogen radicals per molecule, (c) a metal catalyst for the addition reaction of silicon-bound vinyl groups with silicon-bound hydrogen atoms, and (d) an alcohol-group-containing organopolysiloxane and/or a polyether-group-containing organopolysiloxane. The curable composition can be coated on a base material and cured to form an adhesive film.

It is the object of the present invention to provide an improved silicone dental impression material which has a high wettability in conjunction with a low water sorption value.

This problem is solved, according to the invention, by a silicone rubber dental impression material with a component for increasing the wettability, (a) wherein said component for increasing the wettability

(1) is a silicone polyether molecule with at least one hydrophobic silicone moiety and at least one hydrophilic polyether moiety,

(2) is present in an amount of 0.1 to 10 wt.-%, based on the weight of the mixture of all components, and

(3) has a number average molecular weight of 300 to 3000, and

(b) wherein the silicone dental impression material is of the addition type and has a contact angle less than 35 degrees.

With this type of an additive the contact angle can be lowered to values as low as 10 to 15°, while the water sorption is not substantially increased.

The most preferred silicone dental impression material is of the addition type. It is obtained by reacting (a) a silicone having olefinic groups with (b) a silicone having silane hydrogen in the presence of (c) a catalyst, (d) a filler and (e) optionally a plasticizer. It is supplied in a two-pack system, whereby components (b) and (c) are kept seperate from each other. For details US patent 4 035 453 is pointed out for disclosure

purposes.

Component (a) is preferably a diorganopolysiloxane having terminal units that are triorganosiloxy groups in which at least one of the three organic radicals is a vinyl group. Preferred diorganopolysiloxanes of this type are those of the general formula

$$CH_2=CH(-\overset{R}{\underset{R}{\overset{|}{\underset{|}{Si}}}}-O)_n-\overset{R}{\underset{R}{\overset{|}{\underset{|}{Si}}}}-CH=CH_2$$

in which each R denotes an unsubstituted or substituted monovalent hydrocarbon radical, preferably free of aliphatic multiple bonds, and n denotes an integer. At least 50% of the radicals R are preferably methyl radicals, and examples of other suitable radicals R are ethyl, phenyl and 3,3,3-trifluoropropyl radicals. The value of n should be such that the polymer has a viscosity at 25 °C of from 500 to 300,000 m Pas (cP), preferably not more than 100,000 m Pas (cP).

Component (b) is preferably an organopolysiloxane having at least three Si-bonded hydrogen atoms per molecule. Suitably, this organopolysiloxane contains from 0.01 to 1,7% by weight of Si-bonded hydrogen atoms, and the silicone valencies not satisfied by hydrogen atoms, or siloxane oxygen atoms are satisfied by unsubstituted or substituted monovalent hydrocarbon radicals free of aliphatic multiple bonds, at least 50 percent of the hydrocarbon radicals bonded to silicone atoms carrying hydrocarbon atoms being methyl radicals. It is present in an amount so that 0.75 to 5 g-atoms Si-H are present per g-molecule of the olefinic group.

Component (c) is a catalyst that will promote the addition of Si-bonded hydrogen to vinyl groups at room temperature. The most suitable catalysts are platinum and its compounds, such as chloroplatinic acid or a platinum siloxane complex. The platinum and platinum compounds are preferably used in amounts of from 5 to 500 ppm, preferably from 5 to 100 ppm, calculated as Pt and relative to the total weight of all components.

The filler may be any conventional reinforcing or non-reinforcing filler. It may be calcium carbonate, quartz powder, diatomaceous earth, titanium dioxide, zirconium silicate, aluminum silicate, zinc oxide, plaster of paris, so-called "molecular sieves" and talc. Preferably, the fillers are used in amounts of from 5 to 90% by weight, relative to the total weight of components.

Also plasticizers may be present, notably organopolysiloxanes free of aliphatic multiple bonds and Si-bonded hydrogen, for example dimethylpolysiloxanes that are liquid at room temperature and are end-blocked by trimethylsiloxy groups. Other additives, such as pigments, dye-stuffs or flavoring substances may be used.

It is also possible to use a silicone dental impression material of the condensation type as described in US patent 3 897 376, to which reference is made for disclosure purposes. It consists of a base paste and an accelerator or catalyst. The base paste contains a low molecular weight silicone liquid such as dimethylsiloxane with reactive hydroxyl groups and a filler as described above. The accelerator may be in liquid form or, with a content of a thickening filler, in paste form. It comprises an orthoalkyl silicate and a tin octoate, again a plasticizer or other additives as described above may be present.

The additive for increasing the wettability is a silicone compound with one or several hydrophilic polyether moieties. It is possible to have the polyether moiety bonded to one of the silicone components present in the base material, i.e. components (a) or (b) in the additive tpye material or the silicone liquid in the condensation type material. It is most preferred, however, to use a low molecular weight plasticizer with one or several silicone moieties and one or several polyether moieties in the molecule. A number of preferred structural types shall now be given:

$$PS\text{-}A\text{-}PE \qquad (1)$$

$$PS\text{-}(A\text{-}PE)_2 \qquad (2)$$

$$PS\text{-}(A\text{-}PE)_3 \qquad (3)$$

$$PS\text{-}A\text{-}PE\text{-}(A\text{-}PS\text{-}A\text{-}PE)_n\ A\text{-}PS \qquad (4)$$

$$PE\text{-}(A\text{-}PS\text{-}A\text{-}PE)_m\text{-}A\text{-}PS \qquad (5)$$

$$PE\text{-}(A\text{-}PS\text{-}A\text{-}PE)_m\text{-}A\text{-}PS\text{-}A\text{-}PE \qquad (6)$$

wherin PS refers to a polysiloxane moiety, PE to a polyether moiety, A to a bridge group or a single bond, n to an integer of 0 to 10 and preferably 0 to 5 and especially 0, 1 or 2, and m to an integer of 1 to 5 and especially 1, 2 or 3. Each terminal PS or PE carries an end group.

Formulae (1) to (6) give only examples out of a large variety of possible structural types, structure (1) being most preferred. In all these and in other structural types the following characteristics should be satisfied.

The number average molecular weight of the additive should preferably be 300 to 3000 and more preferably 400 to 2000 and most preferably 500 to 1500.

The polysiloxane moieties PS have preferably repeating units of the following formula

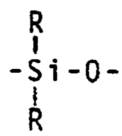

wherein R may refer to a hydrocarbon rest, preferably $C_{1\text{-}6}$-alkyl, for example methyl. The various R residues may be different from each other. The end groups are either R or $-SiR_3$. Depending on the structural types one or several of the R residues may be a bridge group A which is bonded to a PE-rest or in case of a branched PS to another rest PS.

The polyether moieties PE have repeating units of the following formula

-alkylene-O-

wherein alkylene refers to a $C_{2\text{-}4}$-alkylene group and preferably a $C_{2\text{-}3}$-alkylene group, notably $-CH_2\text{-}CH_2-$, $-CH_2\text{-}CH_2\text{-}CH_2-$, $-CH_2\text{-}CH(CH_3)-$, $-CH_2\text{-}CH_2\text{-}CH_2\text{-}CH_2-$. One or several types of alkylene group may be present. Several types of alkylene group, notably $-CH_2\text{-}CH_2-$ and $-CH_2\text{-}CH(CH_3)-$ may be present in block or random arrangement. The end groups may be OH or O-alkyl or -O-CO-alkyl or $-O\text{-}Si(CH_3)_3$.

If A is a bridge group, it may be -O- or, more preferably, a $C_{1\text{-}4}$-alkylene group.

The molar ratio of the groups

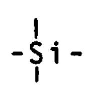

in the PS-moiety and optionally in an end group to alkylene groups in the PE-moiety and optionally in A may be in the range 50:50 to 10:90, preferably 40:60 to 10:90, especially 30:70 to 10:90 and most preferably 25:75 to 15:85.

Further, the molecule must have a strongly hydrophobic portion and a strongly hydrophilic portion. To this end at least one of the PS residues should be a sufficiently long block with at least two and preferably at least three Si-units and at least one of the PE residues should be a sufficiently long block with at least three and preferably at least four alkylene-O-groups. Preferably the above defined molar ratios, which have been given for the entire molecule, should also hold for the strongly hydrophobic and the strongly hydrophilic blocks of the molecule.

In the preferred structural type (1) moiety PS has preferably a structure of the following formula

$$(CH_3)_3SiO-[(CH_3)_2Si-O]_m-Si(CH_3)-(O-Si(CH_3)_2)_{m'}-CH_3$$

wherein m and m' are the same or different and preferably 0 to 5 and especially 0 to 3 and most preferably 0,1 or 2. For example m may be 0 and m' 1.

Many compounds satisfying the above requirements for the additives of the invention are commercially available. Others may be easily prepared according to methods stated in W. Noll, Chemie und Technologie der Silicone, Vlg. Chemie, Weinheim, 1960 and later editions, Section 7.4.4.

The amount of the additive for increasing the wettability is preferably 0.1 to 10 weight-% and especially 0.2 to 6 weight-% and most preferably 0.3 to 3 weight-%. It may be added to one of the packs or to both.

If the silicone polyether additive has terminal hydroxyl groups, it creates shelf stability problems, since it contains active hydrogen, which is abstracted and poisons the reaction so that the shelf life of the resulting composition is deleteriously affected. However, this can be overcome by acetylation, alkylation or silylation of the active hydrogen group, such as hydroxyl.

Furthermore, it was found that the introduction of the silicone-polyether additive tends to aggravate another problem. This is the stability problem caused by moisture. In a most preferred embodiment of this invention, this problem is overcome by water-absorbing inorganic fillers such as calcium sulfate hemihydrate, anhydrous calcium sulfate, calcium chloride, sodium sulfate and potassium sulfate, and absorbing compounds such as zeolites, molecular sieves, alumina and other similar absorbing and adsorbing compounds. These fillers may be used exclusively or in combination with other conventional inorganic fillers. The addition of these fillers alleviate the stability problems caused by water and any active hydrogen in the additive so that the removal of the active hydrogen in the additive may be avoided. This water-absorbing filler may amount to 5 to 100 and preferably 5 to 50 and especially 5 to 30 weight-% of the total amount of the inorganic filler. The mixed product should contain 1 to 15 and especially 1 to 10 weight-% of the water-absorbing filler.

The invention is now described with reference to examples.

Example 1

(i) 2050 g of α,ω-divinylpolydimethylsiloxane with a viscosity of about 25000 mPa s and 880 g of silicondioxide filler of less than 0.1% water content and a particle size distribution between about 0.02 and 30 µm are mixed in a planetary mixer at a reduced pressure of about 80 to 100 mbar until a homogeneous paste is obtained. At the same pressure, 100 ppm of platinum in the form of hexachloroplatinic acid hexahydrate, dissolved in as little absolute ethanol as possible, is admixed as quickly as possible to obtain a smooth paste with a density of 1.1 to 1.2 g/ml.

(ii) 68 g of polymethylsiloxane, having 4 to 5 mmole Si-H/g and a viscosity of below 40 mPa s, 617 g of α,ω-divinylpolydimethylsiloxane with a viscosity of about 25000 mPa as and 282 g of silicondioxide filler of less than 0.1% water content and a particle size distribution between about 0.02 and 30 µm are mixed in a planetary mixer at a reduced pressure of about 80 to 100 mbar until a homogeneous paste is obtained. This paste is subdivided into five samples of 10 g each. To four of these samples, 0.2, 06, 1.2, 1.8 g of the additive of the following formula are added. One sample is left unmodified for comparative purposes.

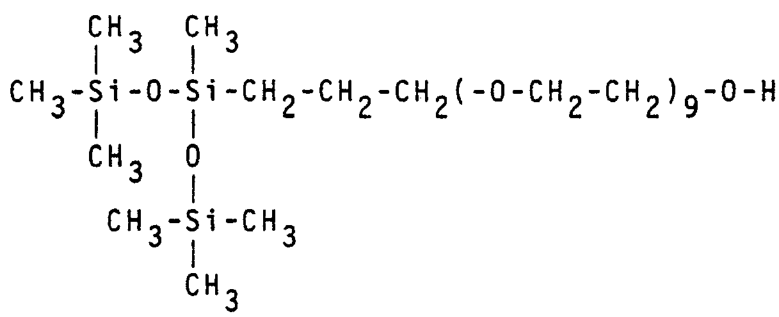

(iii) Each of the five pastes obtained in step (ii) is mixed with 10 g of the paste obtained in step (ii) for 45

s. The mixtures are cured in cylindrical molds in a water bath of 32° C, the bottom and top surface being covered with PE-sheets and the whole clamped together with flat glass plates. The contact angle with water was measured according to the method stated in The Journal of Prosthetic Dentisting 42, 342-347 (1979). The water uptake was measured as percent weight gain after storage during 24h in 93% rel. humidity. The results are given in table I. Care is taken not to touch the cured samples before contact angle and water uptake are measured.

TABLE I

| amount of additive in cured sample (%) | contact angle (degrees) | water uptake (%) |
|---|---|---|
| 0 | 90 | 0.0 |
| 1 | 35 | 0.2 |
| 2,9 | 20 | 0.3 |
| 5,6 | 17 | 0.3 |
| 8,2 | 16 | 0.4 |

Example 2

A paste is prepared in accordance with example 1(ii) except that 0.9 g of the additive were used and further 0.5 g of molecular sieve or calcium sulfate or sodium sulfate as siccative were thoroughly mixed with the paste. Then, 10 g of the paste obtained in Example 1(i) were added and mixed for 45 s. The bench set time, i.e. the time from start of mixing at 23° C until mixture does not stick any more to the spatula (cohesion exceeds adhesion to spatula, beginning of plastic phase) was measured. The results are stated in table II.

TABLE 2

| sample | amount of additive in cured sample | type of siccative | bench set time (min) |
|---|---|---|---|
| 1 | 0 | none | 7.5 |
| 2 | 4.3 | none | 15 |
| 3 | 4.3 | molecular sieve[1)] | 8 |
| 4 | 4.3 | calcium sulfate[2)] | 8.5 |
| 5 | 4.3 | sodium sulfate[2)] | 9 |

1) Union Carbide Type 3A

2) dried for 15 h at 110°C

Samples 1 to 5 were stored at 70° C for 14 days. At the end of that time they were tested for bench set. Sample 2 would not polymerize to a rubbery compound while samples 1 and 3 to 5 set to a rubbery mass in less than about 10 minutes to yield a product complying with the provisions of ADA Spec. 19.

## Claims

**1.** A silicone rubber dental impression material with a component for increasing the wettability,

a. characterized in that the component for increasing the wettability

(1) is a silicone polyether molecule with at least one hydrophobic silicone moiety and at least one hydrophilic polyether moiety,

(2) is present in an amount of 0.1 to 10 wt.-%, based on the weight of the mixture of all components, and

(3) has a number average molecular weight of 300 to 3000,

b. characterized in that the silicone dental impression material is of the addition type and has a contact angle less than 35 degrees.

**2.** A silicone rubber dental impression material according to Claim 1, characterized by a water-absorbing or water-adsorbing inorganic filler, preferably calcium sulfate hemi-hydrate or a molecular sieve.

## Revendications

**1.** Matériau pour empreinte dentaire du type caoutchouc silicone, comportant un composant destiné à augmenter la mouillabilité,

a. caractérisé en ce que le composant destiné à augmenter la mouillabilité

(1) est une molécule de silicone-polyéther comportant au moins un fragment silicone hydrophobe et au moins un fragment polyéther hydrophile,

(2) est présent en une quantité de 0,1 à 10 % en poids par rapport au poids du mélange de tous les composants, et

(3) a une masse moléculaire moyenne en nombre de 300 à 3000,

b. caractérisé en ce que le matériau pour impression dentaire à base de silicone est du type par addition et a un angle de contact inférieur à 35°.

**2.** Matériau pour empreinte dentaire à base de caoutchouc silicone selon la revendication 1, caractérisé par une charge minérale absorbant ou adsorbant l'eau, de préférence le sulfate de calcium semi-hydraté ou un tamis moléculaire.

## Patentansprüche

**1.** Dentales Abdruckmaterial aus Silikongummi mit einem Bestandteil zur Erhöhung der Benetzbarkeit,
   a. dadurch gekennzeichnet, daß der Bestandteil zur Erhöhung der Benetzbarkeit
      (1) ein Silikonpolyethermolekül ist mit mindestens einem hydrophilen Poyletheranteil,
      (2) vorhanden ist in einem Anteil von 0,1 bis 10 Gew.-%, bezogen auf das Gewicht von allen Komponenten der Mischung und
      (3) ein durchschnittliches Molekulargewicht (Zahlenmittel) von 300 bis 3000 hat
   b. dadurch gekennzeichnet, daß das dentale Abdruckmaterial ein Material vom Additionstyp ist und einen Randwinkel von weniger als 35 Grad besitzt.

**2.** Dentales Abdruckmaterial aus Silikongummi nach Anspruch 1, dadurch gekennzeichnet, daß es einen Wasser absorbierenden oder Wasser adsorbierenden anorganischen Füllstoff, vorzugsweise Kalziumsulfathalbhydrat oder ein Molekularsieb enthält.